# NEUE EUROPÄISCHE PATENTSCHRIFT

(11) **EP 2 318 384 B2**
(45) Veröffentlichungstag und Bekanntmachung des Hinweises auf die Entscheidung über den Einspruch: **13.11.2019**
(45) Hinweis auf die Patenterteilung: 25.01.2012
(21) Anmeldenummer: 09775608.4
(22) Anmeldetag: 24.08.2009
(51) Int. Cl.: C07D 301/26, C07C 29/62

(54) **VERFAHREN ZUR HERSTELLUNG VON EPICHLORYDRIN AUS GLYZERIN**
PROCESS FOR PREPARING EPICHLOROHYDRIN FROM GLYCEROL
PROCÉDÉ DE PRÉPARATION D'ÉPICHLORHYDRINE À PARTIR DE GLYCÉROL

(30) Priorität: 25.08.2008 AT 13092008
(43) Veröffentlichungstag der Anmeldung: 11.05.2011
(73) Patentinhaber: Kanzler, Walter, 8010 Graz (AT)
(72) Erfinder: Kanzler, Walter, 8010 Graz (AT)
(74) Vertreter: Wildhack & Jellinek
(86) Internationale Anmeldenummer: PCT/AT2009/000326
(87) Internationale Veröffentlichungsnummer: WO 2010/022422

(56) Entgegenhaltungen:
- WO-A-2005/021476
- WO-A-2005/054167
- WO-A1-2006/100314
- WO-A1-2008/128014
- DE-A1-102008 007 622

## Beschreibung

Die Erfindung betrifft *ein Verfahren gemäß dem Oberbegriff des **Anspruches** 1.*

Bei der Spaltung und Umesterung von Triglyzeriden wird Glyzerin in einer Menge von etwa 10 % der eingesetzten Triglyzeride gebildet, das wegen der hohen Reaktivität seiner drei OH-Gruppen für chemische Synthesen der verschiedensten Art bestens geeignet ist.

Die DE 197308 B beschreibt ein Verfahren, in dessen Verlauf das Glyzerin mit gasförmigem Chlorwasserstoff unter Überdruck - mittels Essigsäure oder Propionsäure katalysiert - zu Dichlorpropanol reagiert, wobei sich ein Gleichgewicht mit etwa 75 % Umsatz einstellt.

Die DE 1 075 103 B beschreibt ein Verfahren, bei dem gegebenenfalls in Anwesenheit von schwefeliger Säure in einer einstufigen Destillation ein Azeotrop aus Wasser und Dichlorpropanol abdestilliert und im Anschluss daran mit Kalkmilch verseift wird und danach in einem horizontalen Destillationsrohr ein *Azeotrop* aus Epichlorhydrin und Wasser abgezogen wird. Bei diesem Verfahren kann Dichlorpropanol nur mit einer Ausbeute von etwa 10 % des eingesetzten Glyzerins gewonnen werden, und in der Verseifung entstehen große Mengen Kalziumchlorid in verunreinigter Form als umweltbelastender Rückstand.

Die WO 2005/021476 A1 beschreibt die Chlorierung von Glyzerin und Monochlorpropandiol in einer Reaktorkaskade, katalysiert durch Essigsäure, unter Abtrennung eines Azeotropes, bestehend aus Reaktionswasser und einem Teil des produzierten Dichlorpropanols . Die Hauptmenge des Dichlorpropanols wird nach einer weiteren Destillation wieder in den Reaktor rückgeführt und nur das Sumpfprodukt, bestehend aus den Rückständen wird ausgeschleust.

Dieses Verfahren hat den Nachteil, dass im Kopfprodukt der Destillation noch größere Mengen an Chlorwasserstoff enthalten sind und entweder das abdestillierte Wasser mit dem Chlorwasserstoff bei starkem Unterdruck und niedriger Temperatur nur in einer Kälteanlage kondensiert werden kann oder das im Reaktor gebildete Dichlorpropanol nur mit einem erheblichen Rücklauf an Wasser in den Kopf der Rektifikationskolonne als Azeotrop mit nur ca. 23 % Gehalt an Dichlorpropanol gewonnen werden kann, was einen enormen Energieverbrauch bei der Rektifikation verursacht.

Die als Katalysator eingesetzte Essigsäure wird wegen ihres hohen Dampfdruckes in der Rektifikation aus dem Reaktionsgemisch ausgetragen, erhöht bei der nachfolgenden Verseifung den Alkali-Verbrauch und verunreinigt die Salzlösung.

Die WO 2005/054167 A1 beschreibt Verfahren zur Chlorierung verschiedener Qualitäten von Glyzerin mit Verunreinigungen aus den Herstellungsprozessen von Glyzerin. Als Katalysator wird Essigsäure oder eine andere Karbonsäure, wie Adipinsäure oder deren Derivate vorgeschlagen. Der Einsatz von verunreinigten Glyzerinen führt meist zur Verschlechterung der Ausbeuten und zur Bildung von großen Mengen an Destillationsrückständen, die in Verbindung mit chlorierten Komponenten nur kostspielig zu entsorgen sind und die daher besser vor der Chlorierung abgetrennt werden.

Aufgabe der vorliegenden Erfindung ist es nun, die Nachteile der aus dem Stand der Technik bekannten Verfahren weitestgehend zu vermeiden, und den Energieeinsatz sowie die Menge an zu entsorgenden Rückständen auf ein Mindestmaß zu reduzieren.

Die erfindungsgemäße Aufgabe besteht also darin, ein Verfahren zu schaffen, bei welchem Epichlorhydrin durch Chlorierung von Glyzerin mit Chlorwasserstoff und anschließende Verseifung hergestellt wird.

Um die oben genannten Nachteile zu vermeiden, ist das neue Verfahren gemäß dem eingangs genannten Oberbegriff des **Anspruches** 1 *durch die kennzeichnenden Merkmale des Anspruchs 1 gekennzeichnet.*

Die Verseifung des Dichlorpropanols erfolgt gemäß dem **Anspruch** 2 vorteilhafter Weise in zwei Stufen mit wässeriger Natronlauge, wobei in der ersten Stufe die Natronlauge in stöchiometrischem Verhältnis zugegeben wird und der Großteil des dadurch gebildeten Epichlorhydrins in einer Rektifikationskolonne abdestilliert wird und in einer zweiten Stufe unter Nachdosierung von Natronlauge das restliche Dichlorpropanol praktisch vollständig zu Epichlorhydrin umgesetzt und in einer Strippkolonne, vorzugsweise Rektifikationskolonne, abdestilliert wird und die gebildete wässrige Natriumchlorid-Lösung, gegebenenfalls nach Durchlaufen einer notwendigen Nachreinigungsstufe, als Rohstoff für die Chlor-Alkali-Elektrolyse zur Wiedergewinnung von Natronlauge eingesetzt wird.

Es wurde überraschenderweise gefunden, dass - wie im **Anspruch** 3 geoffenbart - der Einsatz von Oxalsäure als Katalysator in der Blasensäule die höchsten Reaktionsgeschwindigkeiten für den Umsatz von Chlorpropandiol zu Dichlorpropanol erbringt, wodurch die Ausbeute pro Zeiteinheit vergleichsweise wesentlich erhöht wird und gleichzeitig die Menge an gebildeten Rückständen und Nebenprodukten wesentlich niedriger ist, als bei den bisher bekannt gewordenen Epichlorhydrin-Herstellungsverfahren.
Im **Anspruch** *4* sind die für das Verfahren günstigsten Konzentrationen der Oxalsäure in der Blasensäule im Bereich von 0,5 bis 10 Gew-% angegeben.

Der Einsatz einer Blasensäule als Reaktor für die Chlorierung ist besonders vorteilhaft, da der statische Druck am Sumpf der Blasensäule, die beispielsweise 5 bis 15m hoch ist, den Partialdruck des Chlorwasserstoffs erhöht, wobei die auf diese Weise erreichte höhere Löslichkeit desselben in der Reaktorflüssigkeit die Reaktionsgeschwindigkeit beträchtlich steigert.

Es hat sich auch gezeigt, dass bei einem Druck am Kopf der Kolonne von 1 bar absolut und bei einer Flüssigkeitshöhe von über 5 m bei Reaktionstemperaturen zwischen 100 und 130 °C am Kopf der Blasensäule praktisch fast kein Chlorwasserstoff mehr entweicht.

Es hat sich im Rahmen der Erfindung weiters als vorteilhaft erwiesen, diese geringe verbleibende Menge an Chlorwasserstoff vom Kopf der Blasensäure in einer dort anschließenden Waschkolonne mit dem frisch zugeführten, voll beladungsfähigen Glyzerin auszuwaschen um den Chlorwasserstoff-Gehalt am Kopf und den Gehalt an organischen (Chlor-)Verbindungen u. dgl. so gering wie möglich zu halten.

Gleichzeitig ist es für die rasche Umsetzung wichtig, am Kopf der Blasensäule so viel und so rasch Flüssigkeit abzuziehen und in die nachfolgende Destillationseinheit einzuspeisen, dass das Gleichgewicht der Reaktion möglichst weit nach rechts zum Dichlorpropanol hin verschoben wird. Da die Chlorierung zum Monochlorpropandiol etwa 20 mal so schnell verläuft wie die Reaktion zum Dichlorpropanol, ist es weiters wichtig, die Wasser-Konzentration in der Blasensäule möglichst niedrig zu halten und aus dem Reaktor am besten über eine Vakuum-Rektifikationskolonne, bei 100 bis 200 mbar absolut die gebildeten Reaktionsprodukte abzutrennen.

Es ist günstig bzw. gegebenenfalls notwendig, einen kleinen Teil des Sumpfproduktes dieser Rektifikationskolonne über eine weitere Vakuum-Rektifikation zu leiten, um Polymere und Nebenprodukte im Sumpf auszuschleusen.

Durch diese Art der Reaktionsführung wird Dichlorpropanol mit hoher Reinheit gewonnen, das bei der nachfolgenden Verseifung keine Nebenprodukte bildet. Dadurch kann die Verseifung vollständig durchgeführt werden und das Azeotrop, bestehend aus Epichlorhydrin und Wasser kann in den - erfindungsgemäß günstiger Weise vorgesehenen beiden nachfolgenden - Strippkolonnen vollständig abgetrennt werden, so dass die verbleibende Natriumchlorid-Lösung hohe Reinheit und eine Konzentration von mehr als >30Gew-% aufweist, je nach Konzentration der für die Verseifung eingesetzten Natronlauge.

*Erfindungsgemäß* ist vorgesehen, dass der Blasensäule die Einsatzstoffe Glyzerin und Salzsäure sowie aus der Rektifikationskolonne abgezogenes Glyzerin, Di- und Monochlorpropanol vom Boden her zugeführt werden, womit Gleichstrom der Reaktanden erzielt wird.

*Erfindungsgemäß* ist vorgesehen, dass ein Teilstrom des Sumpfproduktes aus der der Blasensäule folgenden Rektifikationkolonne für die Abtrennung des Dichlorpropanols in eine weitere (Vakuum-)Rektifikationskolonne geleitet wird, in welcher Chlorpropandiol von den Hochsiedern, wie insbesondere Polymeren, Diglyzeriden u. dgl. abgetrennt und über Kopf abdestilliert wird, und wo die Hochsieder als Destillationsrückstand als Sumpf anfallen.

### Referenzbeispiel:

Glyzerin mit einer Konzentration von 99,5 Gew-% mit 0,4 Gew-% Wasser und 0,1 Gew-% Fettsäureresten wird mit einem Massenstrom von 2000 kg/h in die Blasensäule K10 an deren Sumpf gemeinsam mit 1560 kg/h Chlorwasserstoff und dem Retourlauf aus den Destillations- bzw. Rektifikationskolonnen K14 und K18 aufgegeben. Die Blasensäule K10 hat z.B. eine Höhe von ca. 15 m und der Füllstand der Flüssigkeit in derselben liegt bei etwa 10 m. Der Betriebsdruck in der Blasensäule ist 1 bar abs. und die Temperatur wird dort auf zwischen 120 und 130 °C gehalten. Der aufgegebene Chlorwasserstoff wird beinahe zu 100% umgesetzt, das Glyzerin wird zu 99 % zu Monochlorpropandiol umgesetzt und die Konzentration des Dichlorpropanols beträgt im Sumpf etwa 12 Gew-%.

Vom Kopf der Blasensäule K10 werden etwa 25.000 kg/h Flüssigkeit abgezogen und im nachfolgenden Stripper K14 werden 1160 kg/h Wasser und etwa 2.346 kg/h Dichlorpropanol über Kopf abgezogen und deren Sumpfprodukt wird zu mehr als 90 % in die Blasensäule K10 rückgeführt, während die restlichen 10% des Sumpfproduktes der Vakuum-Rektifikation K18 zugeführt, an deren Kopf nicht umgesetztes Monochlorpropandiol und restliches Dichlorpropanol abgezogen werden, während vom Sumpf etwa 45 kg/h Nebenprodukte, vermischt mit etwa 25 kg Monochlorpropandiol abgezogen werden.

Es werden auf diese Weise etwa 98 % der theoretischen Ausbeute an Dichlorpropanol aus der Chlorierungsstufe abgezogen und der Verseifung zugeführt.

Die Verseifung des Dichlorpropanols wird mit ca. 2.750 kg/h 30 gew-%iger Natronlauge durchgeführt und in der Rektifikationskolonne K20 werden am Kolonnenkopf ca. 1.900 kg/h Epichlorhydrin in Form eines wässrigen Azeotropes mit etwa 26 Gew-% Wassergehalt abgezogen, welches bei der Kondensation in eine wässerige Phase und in eine Epichlorhydrin-Phase zerfällt, wobei die wässerige Phase wieder als Rücklauf aufgegeben wird.

Im Sumpf der Kolonne K20 wird die über 30 gew-%ige Natriumchlorid-Lösung mit etwa 50 kg/h Dichlorpropanol und Epichlorhydrin-Resten abgezogen, und unter Zusatz von weiteren 43 kg/h 30 Gew-% iger Natronlauge wird die Verseifung in der Mischstufe B26 komplettiert, und die dort gebildete Salzlösung wird der weiteren Strippkolonne K30 zugeführt.

Am Kopf dieser Kolonne K30 wird ebenfalls ein Azeotrop aus dem restlichen Epichlorhydrin und Wasser abgezogen und im Sumpf werden etwa 4.350 kg/h etwa 28 gew-%ige Natriumchlorid-Lösung mit unter 5 kg/h Gehalt an Chlorkohlenwasserstoffen abgezogen, die dann noch einer an sich bekannten Behandlung mit Ozon oder WasserstoffPeroxid unterzogen werden und schließlich als Rohstoff in der Chlor-Alkali-Elektrolyse eingesetzt werden, um daraus die für die Verseifung vorgesehene Natronlauge (NaOH) zu gewinnen.

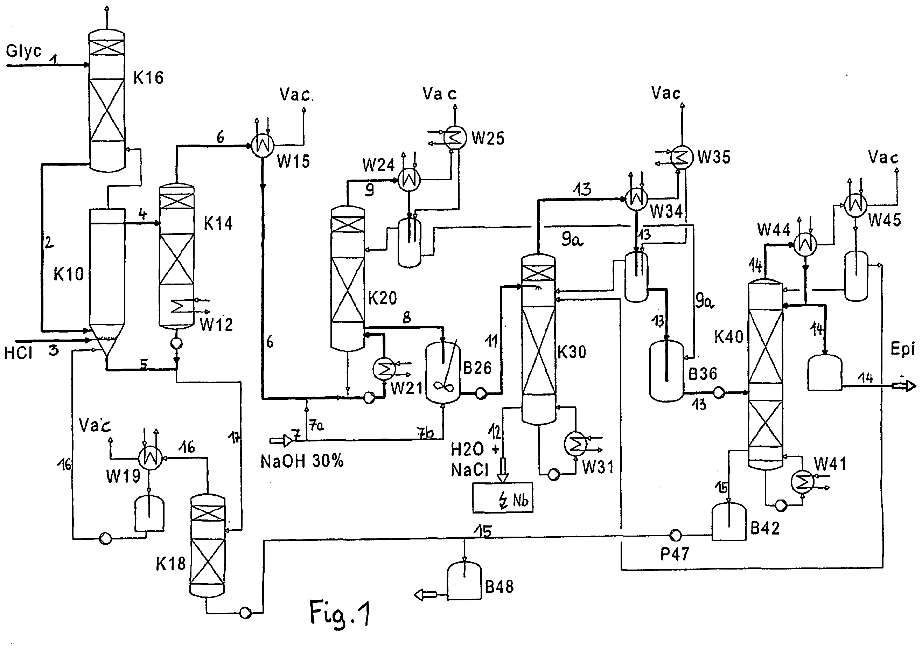

Anhand der Zeichnung wird das erfindungsgemäße Verfahren näher erläutert:
Über den vom Kopf der Kolonne K10 aus dem mit geringen Mengen Rest-Chlor beaufschlagten Wäscher K16 wird - zuerst über die Leitung 1 frisch zugeführtes - im Wäscher K16 mit geringen Mengen Chlorwasserstoff beladenes Glyzerin über die Leitung 2 zum Boden des Blasenreaktors K10 geführt und in diesen zusammen mit frischem, über Leitung 3 zugeführten Chlorwasserstoff eingebracht. Weiters wird dort als Sumpf in der der Blasensäule K10 folgenden Destillationskolonne K14 anfallendes Dichlorpropan, Glyzerin u. dgl. über Leitung 5 eingebracht und weiters wird über Leitung 16 noch das Dichlorpropanol enthaltende Kopfprodukt einer mit einem Teilstrom des Sumpfproduktes der Destillationskolonne K14 über Leitung 17 versorgten nebengeschalteten Destillationskolonne K18 für die Aufarbeitung von anfallenden Restmengen und Rückständen eingebracht.

Das Kopfprodukt der Blasensäule K10 wird über die Leitung 4 in die erste, schon oben genannte Vakuum-Destillationskolonne K14 eingebracht und von deren Kopf wird das Dichlorpropanol als erwünschtes Produkt enthaltende Gemisch über Leitung 6 nach Zugabe von 30 Gew-% Natronlauge (NaOH) über die Leitungen 7, 7a in den Sumpf einer weiteren Vakuum-Rektifikationskolonne K20 eingebracht. Über deren Kopf wird über Leitung 9 das dort gebildete Epichlorhydrin-Wasser-Gemisch abgezogen.

Aus dem Sumpf der Rektifikationskolonne K20 wird Natriumchlorid-Lösung und restliches, nicht umgesetztes Dichlorpropan ausgetragen und es wird nach Durchlaufen der Leitung 8 in einer Mischeinheit G26 mit einer über die Leitung 7b zugeführten - dem aktuellen Gehalt an Dichlorpropan entsprechenden molaren - Menge an 30 %-ig wässriger NaOH versetzt und umgesetzt.

Danach erfolgt über Leitung 11 die Einspeisung des dort gebildeten Gemisches in eine erste Strippkolonne K30, aus welcher von deren Sumpf über Leitung 12 wässrige Natriumchlorid-Lösung abgezogen wird, welche einer Sole-Nachbehandlung NB mit Chlor-Alkali-Elektrolyse zugeführt wird.

Vom Kopf der Vakuums-Strippkolonne K30 wird Roh-Epichlorhydrin über die Leitung 13 abgezogen und in einen Behälter B36, in welchen auch ein Teilstrom des Epichlorhydrin enthaltenden, schon am Kopf der vorherigen Kolonne K20 abgezogenen Gemisches über Leitung 9a zugeleitet wird, gesammelt. Von dort gelangt über die Leitung 13 das Roh-Epichlorhydrin hier in eine Vakuum-Destillierkolonne K40, über deren Kopf schließlich über Leitung 14 das gewünschte reine Epichlorhydrin abgezogen wird.

Aus dem Sumpf der eben genannten Vakuum-Destillierkolonne K40 werden schließlich über Leitung 15 die dort zurückgebliebenen Sumpfprodukte in die eingangs oben erwähnte Vakuum-Destillationskolonne K18 geführt, von deren Kopf aus über die Leitung 16 der Sumpf der Blasensäule K10 mit dem dort abgezogenen Gemisch versorgt wird, womit der Kreislauf geschlossen ist.

Die wesentlichen Vorteile des neuen, mit einem Blasenreaktor und einer davon getrennten Destillation als wesentliche Kern-Apparatur arbeitenden Verfahren liegen insbesondere in der neuen Gleichstromführung von Glyzerin und Chlorwasserstoff im Blasenreaktor sowie in der, insbesondere durch den laufenden Abzug des gebildeten Dichlorpropans und dann des abzudestillierenden Epichlorhydrins, auf die Produktseite hin verschobenen Gleichgewichts.

Hinzu kommt die überraschende wesentliche Erhöhung der Reaktionsgeschwindigkeit in der Höhe von zumindest 20% im Vergleich zu jedem der bisher bekannten "besten" Verfahren was insbesondere Folge des Einsatzes von Oxalsäure als zweibasige Carbonsäure anstelle der bisher als Katalysator eingesetzten organischen Säuren, wie Essigsäure, Adipinsäure und deren Derivate.

## Patentansprüche

1. Verfahren zur Herstellung von Epichlorhydrin durch Chlorierung von Glyzerin mit Chlorwasserstoff, welches mittels zumindest einer Carbonsäure katalysiert wird, in einem Reaktor und mindestens einer *Rektifikationskolonne* mit anschließender Verseifung des gebildeten Dichlorpropanols mittels Natronlauge, wobei das Glyzerin mit gasförmigem Chlorwasserstoff in einer Blasensäule (K10) chloriert wird, wobei Chlorwasserstoff, Glyzerin und eine aus den - der Blasensäule nachgeschalteten - Rektifikationskolonnen (K14, K18) zurückgeführte Mischung aus Monochlorpropandiol sowie Resten von Glyzerin, Dichlorpropanol und Rückständen dem Sumpf der Blasensäule (K10) zugeführt werden und in Nähe des Kopfes der Blasensäule (K10) die in derselben gebildete Mischung, bestehend aus Monochlorpropandiol, Dichlorpropanol, Wasser, Resten von Glyzerin und Rückständen abgezogen wird und einer ersten Rektifikationskolonne (K14) zugeführt wird, wonach das dort über Kopf abdestillierte Dichlorpropanol und Wasser mit Natronlauge verseift wird,
**dadurch gekennzeichnet, dass** vorgesehen ist, dass, vom Kopf der Blasensäule (K10), dort nicht umgesetzte, geringe Restmengen Chlorwasserstoff abgezogen werden und in einem dem Kopf der Blasensäule (K10) nachgeschalteten Wäscher (K16) mit dem frisch zugeführten Glyzerin absorbiert werden, und dass das auf diese Weise mit Chlorwasserstoff vorbeladene, Chlorwasserstoff enthaltende Glyzerin am Boden bzw. Sumpf der Blasensäule (K10) zusammen mit frischem Chlorwasserstoff zugeführt wird,
wobei der Blasensäule (K10) *die Einsatzstoffe Glyzerin und Salzsäure sowie* vom Sumpf der ersten Rektifikationskolonne (K14) abgezogenes Glyzerin, Di- und Monochlorpropanol vom Boden her zugeführt werden,
und wobei ein Teilstrom des Sumpfproduktes aus der ersten Rektifikationskolonne (K14) zur Abtrennung von dort enthaltenem Monochlorpropanol in eine weitere Rektifikationskolonne (K18) eingebracht wird, mittels welcher über Kopf Monochlorpropandiol von den im Sumpf derselben als Destillationsrückstand verbleibenden Hochsiedem, wie insbesondere Polymere, Diglyzeride u. dgl. abgetrennt und dem Sumpf der Blasensäule (K10) zugeführt wird.

2. Verfahren nach Anspruch 1, **dadurch gekennzeichnet, dass** die Verseifung in zwei Stufen durchgeführt wird, wobei dem aus der *Rektifikationskolonne* (K14) kommenden Dichlorpropanol in einer ersten Stufe Natronlauge (NaOH) in stöchiometrischem Verhältnis zugegeben wird und der Großteil des dort gebildeten Epichlorhydrins in einer Rektifikationskolonne (K20) abdestilliert wird, und in einer zweiten Stufe das aus dieser Rektifikationskolonne (K20) unten abgezogene restliche Dichlorpropanol unter Nachdosierung (B26) von Natronlauge im Wesentlichen vollständig zu Epichlorhydrin umgesetzt wird, dasselbe in einer Strippkolonne (K30), vorzugsweise in einer Rektifikationskolonne, abdestilliert wird und die dort im Sumpf verbleibende wässrige Natriumchlorid-Lösung, nach Durchlaufen einer, gegebenenfalls notwendigen, Nachbehandlungsstufe (Nb) als Rohstoff für die Chlor-Alkali-Elekrolyse zur Wiedergewinnung von Natronlauge eingesetzt wird.

3. Verfahren nach Anspruch 1 oder 2, **dadurch gekennzeichnet, dass** als Katalysator in der Blasensäule (K10) Oxalsäure zum Einsatz kommt.

4. Verfahren nach einem der Ansprüche 1 bis 3, **dadurch gekennzeichnet, dass** die aktuelle Konzentration an Oxalsäure in der Blasensäule (K10) auf 0,5 bis 10 Gew-%, vorzugsweise auf 2 bis 5 Gew-%, jeweils bezogen auf das Gewicht der durch dieselbe geführten Glyzerin-Chlorwasserstoff-Mischung gehalten wird.

## Claims

1. A process for preparing epichlorohydrin by chlorination of glycerol using hydrogen chloride, which is catalysed by means of at least one carboxylic acid in a reactor and at least one *rectification column* with subsequent saponification of the formed dichloropropanol by means of sodium hydroxide, wherein the glycerol is chlorinated in a bubble column (K10) using gaseous hydrogen chloride, wherein hydrogen chloride, glycerol and a mixture consisting of monochloropropanediol and residual amounts of glycerol, dichloropropanol and residues are fed into the sump of the bubble column (K10), said mixture being recycled from the rectification columns (K14, K18) downstream of the bubble column, and the mixture formed in the same which consists of monochloropropanediol, dichloropropanol, water, residual amounts of glycerol and residues, is removed near the head of the bubble column (K10) and fed into a first rectification column (K14), whereafter the dichloropropanol and the water, which are distilled overhead, are saponified using sodium hydroxide solution,
**characterised in that** small residual amounts of hydrogen chloride that have not reacted in the bubble column (K10) are withdrawn from its head, and are absorbed together with freshly fed glycerol in a scrubber (K16) downstream of the head of the bubble column (K10) and **in that** the hydrogen chloride-containing glycerol that is preloaded in this way with hydrogen chloride is fed into the base or sump of the bubble column (K10) together with fresh hydrogen chloride,
wherein *the glycerol and hydrochloric acid* and glycerol, dichloropropanol and monochloropropanol withdrawn from the sump of the first rectification column (K14) are fed into the bubble column (K10) from the base,
and wherein a partial flow of the sump product from the first rectification column (K14) is fed for the separation of monochloropropanol contained therein into a further rectification (K18), by means of which monochloropropanediol is separated via the head from the high boilers, such as polymers, diglycerides and the like, remaining as a distillation residue in the sump thereof and fed into the sump of the bubble column (K10).

2. The process according to claim 1, **characterised in that** the saponification is carried out in two steps, wherein, in the first step, a stoichiometric amount of sodium hydroxide (NaOH) is added to the dichloropropanol obtained from the *rectification column* (K14) and the majority of the epichlorohydrin formed therein is distilled off in a rectification column (K20), and, in the second step, the remaining dichloropropanol withdrawn from the bottom of this rectification column (K20) is substantially completely converted to epichlorohydrin by adding (B26) additional sodium hydroxide, said epichlorohydrin being distilled in a stripping column (K30), preferably in a rectification column, and the aqueous sodium chloride remaining at the sump of the column, after being subjected to an optionally necessary post-treatment step (Nb), is used as a raw material in a chlor-alkali electrolysis for recycling sodium hydroxide solution.

3. The process according to claim 1 or 2, **characterised in that** oxalic acid is used as the catalyst in the bubble column (K10).

4. The process according to any one of claims 1 to 3, **characterised in that** the current concentration of oxalic acid in the bubble column (K10) is maintained at 0.5 to 10% by weight, preferably to 2 to 5% by weight, based in each case on the weight of the mixture of glycerol and hydrogen chloride guided through said bubble column.

## Revendications

1. Procédé de préparation d'épichlorhydrine par chloration du glycérol avec du chlorure d'hydrogène, catalysé par au moins un acide carboxylique, dans un réacteur et au moins une *colonne de rectification* avec saponification ultérieure du dichloropropanol formé au moyen d'une solution d'hydroxyde de sodium, dans lequel le glycérol est chloré avec du chlorure d'hydrogène gazeux dans une colonne à bulles (K10), dans lequel le chlorure d'hydrogène, le glycérol et un mélange récupéré des colonnes de rectification (K14, K18), en aval de la colonne à bulles, consistant en du monochloropropanediol et des restes de glycérol, de dichloropropanol et de résidus sont introduits dans le fond de distillation de la colonne à bulles (K10) et à proximité de la tête de la colonne à bulles (K10) est extrait le mélange, formé des mêmes composés, constitué de monochloropropanediol, de dichloropropanol, d'eau, de restes de glycérol et de résidus, et introduit dans une première colonne de rectification (K14), après quoi le dichloropropanol et l'eau séparés par distillation en tête sont saponifiés avec une solution d'hydroxyde de sodium,
**caractérisé en ce qu'il est** prévu que les faibles quantités résiduelles de chlorure d'hydrogène n'ayant pas réagi sont extraites de la tête de la colonne à bulles (K10) et absorbées avec du glycérol fraîchement amené dans un laveur (K16) placé en aval de la tête de la colonne à bulles (K10), et **en ce que** le glycérol contenant du chlorure d'hydrogène, ainsi préchargé avec du chlorure d'hydrogène ; est introduit dans le bas ou le fond de distillation de la colonne à bulles (K10) avec du chlorure d'hydrogène frais,
*les charges d'alimentation glycérol et acide chlorhydrique* ainsi que le glycérol extrait du fond de distillation de la première colonne de rectification (K14), le di- et le monochloropropanol prélevés au bas de celle-ci étant acheminés vers la colonne à bulles (K10),
et dans lequel on introduit un courant partiel du produit de fond de distillation, provenant de la première colonne de rectification (K14) pour la séparation du monochloropropanol contenu dans celui-ci, dans une colonne de rectification supplémentaire (K18) au moyen de laquelle on sépare par la tête, le monochloropropanediol des composés de haut point d'ébullition restant dans le fond de distillation en tant que résidu de distillation, en particulier des polymères, diglycérides et similaires et on l'introduit dans le fond de distillation de la colonne à bulle (K10).

2. Procédé selon la revendication 1, **caractérisé en ce que** la saponification est effectuée en deux étapes, dans lequel une solution d'hydroxyde de sodium (NaOH) est lors d'une première étape ajoutée selon un rapport stoechiométrique au dichloropropanol provenant de la *colonne de rectification* (K14), et la majorité de l'épichlorhydrine y formée est séparée par distillation dans une colonne de rectification (K20) et, dans une seconde étape, le dichloropropanol résiduel soutiré en bas de la colonne de rectification (K20) est mis à réagir de manière sensiblement complète en épichlorhydrine avec addition supplémentaire (B26) d'hydroxyde de sodium, l'épichlorhydrine est séparée par distillation dans une colonne d'épuisement (K30), de préférence dans une colonne de rectification, et la solution aqueuse de chlorure de sodium restant dans le fond de distillation, après le passage par une étape de post-traitement éventuellement nécessaire (Nb), est utilisée comme matière première pour l'électrolyse chlore-alcali pour la récupération de la solution d'hydroxyde de sodium.

3. Procédé selon la revendication 1 ou 2, **caractérisé en ce que** l'on utilise de l'acide oxalique comme catalyseur dans la colonne à bulles (K10).

4. Procédé selon l'une des revendications 1 à 3, **caractérisé en ce que** la concentration actuelle en acide oxalique dans la colonne à bulles (K10) est maintenue dans l'intervalle allant de 0,5 à 10 % en poids, de préférence de 2 à 5 % en poids, par rapport au poids du mélange glycérol-chlorure d'hydrogène qui y est introduit.
